# FASCICULE DE BREVET EUROPEEN

(11) **EP 2 870 501 B1**
(45) Date de publication et mention de la délivrance du brevet: **26.06.2019**
(21) Numéro de dépôt: 13744690.2
(22) Date de dépôt: 04.07.2013
(51) Int. Cl.: A61B 3/00, A61B 3/14, A61B 3/18, A61B 3/028, G02C 13/00, A61B 3/103, G02B 27/10, A61B 3/113

(54) **DISPOSITIF ET PROCÉDÉ DE MESURE DE RÉFRACTION OCULAIRE OBJECTIVE ET D'AU MOINS UN PARAMÈTRE GÉOMÉTRICO-MORPHOLOGIQUE D'UN INDIVIDU**
VORRICHTUNG UND VERFAHREN ZUR MESSUNG VON OBJEKTIVER OKULARER REFRAKTION UND MINDESTENS EINEM GEOMETRISCH-MORPHOLOGISCHEM PARAMETER EINES INDIVIDUUMS
DEVICE AND METHOD FOR MEASURING OBJECTIVE OCULAR REFRACTION AND AT LEAST ONE GEOMETRIC-MORPHOLOGICAL PARAMETER OF AN INDIVIDUAL

(30) Priorité: 06.07.2012 FR 1201925
(43) Date de publication de la demande: 13.05.2015
(73) Titulaire: Essilor International, 94220 Charenton-le-Pont (FR)
(72) Inventeur: DIVO, Fabien, F-94220 Charenton-Le-Pont (FR); ESCALIER, Guilhem, F-94220 Charenton-Le-Pont (FR)
(74) Mandataire: Jacobacci Coralis Harle
(86) Numéro de dépôt international: PCT/FR2013/051595
(87) Numéro de publication internationale: WO 2014/006341

(56) Documents cités:
- EP-A1- 0 498 363
- WO-A1-2011/058244
- US-A1- 2003 081 173
- US-A1- 2006 290 885

## Description

### DOMAINE TECHNIQUE AUQUEL SE RAPPORTE L'INVENTION

La présente invention concerne de manière générale le domaine des appareils de mesure pour la fabrication de lunettes de correction visuelle.

Elle concerne plus particulièrement un appareil permettant une prise de mesure simultanée d'un paramètre géométrico-morphologique et d'un paramètre de réfraction oculaire dans un ou plusieurs comportements visuels du porteur de lunettes, par exemple en vision de loin (VL) et en vision de près (VP). Plus précisément, l'invention concerne un appareil permettant la mesure de paramètres de monturisation et de paramètres de réfraction oculaire d'un individu.

### ARRIERE-PLAN TECHNOLOGIQUE

Aujourd'hui il est nécessaire de mettre en oeuvre des appareils distincts pour déterminer d'une part les paramètres de réfraction oculaire et d'autre part les paramètres géométrico-morphologiques et notamment les paramètres de monturisation. Dans le langage communément répandu du métier de l'optique ophtalmique, on entend par paramètre « géométrico-morphologique » d'un individu un paramètre géométrique ou morphologique relatif au visage du porteur tel que l'écart inter-pupillaire, l'angle de tête (roulis, tangage, lacet) par rapport à une ligne verticale dans une posture déterminée. On entend par « paramètre de monturisation » un paramètre géométrique ou physionomique relatif au visage du porteur et/ou à la monture de lunettes tels que : la hauteur des yeux par rapport au bord inférieur d'une monture sélectionnée, le vertex (distance entre l'oeil et la face interne du verre de lunettes), l'angle de galbe de la monture, l'angle pantoscopique de la monture.

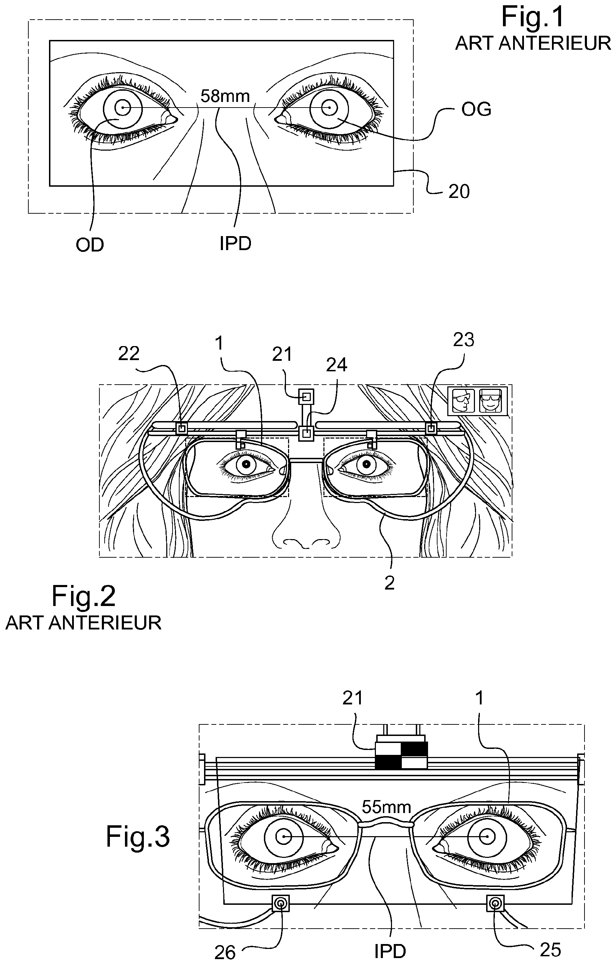

D'une part, la mesure de la réfraction oculaire est effectuée au moyen d'instruments sur table. La réfraction oculaire est généralement mesurée de manière subjective chez le praticien et vérifiée chez l'opticien en monoculaire de manière objective. La figure 1 illustre un écran permettant d'afficher des résultats de mesure des paramètres de réfraction oculaire, la mesure étant effectuée sans lunettes de correction. L'écran affiche en superposition sur une image du visage du sujet, un repère rectangulaire de cadrage 20 et une mesure de la distance inter-pupillaire (IPD). Le même écran peut représenter les mesures de réfraction oculaire pour l'oeil droit (OD) et pour l'oeil gauche (OG) respectivement. L'interface graphique peut par exemple afficher les résultats numériques de mesure de paramètres de réfraction, d'asymétrie du regard, de diamètre pupillaire et d'IPD.

D'autre part, on utilise généralement un appareil sous forme de colonne ou de tablette pour déterminer les paramètres de monturisation. La figure 2 illustre une image d'un individu portant une monture 1 sur laquelle est fixé un clip 2 muni de marqueurs 21, 22, 23, 24. Un traitement d'image permet de déterminer les paramètres de monturisation personnalisés tels que : hauteur du regard (H) par rapport au bord inférieur de la monture, angle pantoscopique de la monture... L'interface graphique représentée sur la figure 2 peut aussi indiquer des résultats de mesure des paramètres de monturisation tels que : la position relative de chaque oeil par rapport au verre considéré, ainsi que le comportement visuel de l'individu lors de la mesure, par exemple l'inclinaison de la tête.

Les différentes étapes de prise de mesure des paramètres de réfraction oculaire et de monturisation demandent au porteur de se déplacer et d'être confronté à au moins deux instruments différents. Pour l'opticien, la succession de ces deux étapes importantes de mesure prend du temps. De plus, la mesure des paramètres de monturisation est généralement effectuée dans une seule posture du sujet et une seule position de vision, la tête du porteur étant droite et le sujet regardant droit devant lui. La mesure des paramètres de monturisation ne prend généralement pas en compte les différentes postures du sujet et/ou différentes conditions de vision. D'autre part, certains instruments requièrent l'utilisation d'une mentonnière et/ou d'un appui frontal afin de déterminer la posture de tête et les conditions de vision du porteur. Ces instruments imposent des contraintes de posture au porteur et ne fournissent pas de mesure en posture et en conditions de vision naturelles, sans contrainte de contact. Les publications WO 2011/058244 A1 et US 2003/0081173 A1 divulguent des dispositifs de mesure de réfraction oculaire et d'au moins un paramètre géométrico-morphologique.

### OBJET DE L'INVENTION

Afin de remédier aux inconvénients précités de l'état de la technique, la présente invention propose de combiner la prise de mesure des paramètres géométrico-morphologiques (notamment de monturisation) avec la mesure de réfraction oculaire pour différentes positions du regard et différentes valeurs de proximité.

Plus particulièrement, on propose selon l'invention un dispositif de mesure de réfraction oculaire objective et d'au moins un paramètre géométrico-morphologique d'un individu selon la revendication 1. Les revendications dépendantes définissent des modes de réalisation additionnels.

L'invention propose également un procédé de mesure de réfraction oculaire objective et d'au moins un paramètre géométrico-morphologique d'un individu selon la revendication 10.

### DESCRIPTION DETAILLEE D'UN EXEMPLE DE REALISATION

La description qui va suivre en regard des dessins annexés, donnés à titre d'exemples non limitatifs, fera bien comprendre en quoi consiste l'invention et comment elle peut être réalisée.

Sur les dessins annexés :
- la figure 1 est une vue d'une interface graphique d'un appareil de mesure de réfraction oculaire selon l'art antérieur ;
- la figure 2 est une vue d'une interface graphique d'un appareil de mesure de paramètres de monturisation selon l'art antérieur ;
- la figure 3 illustre une vue d'une interface graphique d'un appareil de mesure selon un mode de réalisation préféré de l'invention ;
- la figure 4 représente schématiquement en vue de côté un appareil de mesure selon un mode de réalisation de l'invention dans des conditions de mesure en vision de loin ;
- la figure 5 représente schématiquement le même appareil que sur la figure 4, dans des conditions de mesure en vision de près ;
- la figure 6A représente schématiquement un système d'éclairage et un système de capture d'image permettant la mesure simultanée de la réfraction et des paramètres de monturisation suivant une première variante ; la figure 6B représente schématiquement un système d'éclairage et un système de capture d'image suivant une deuxième variante ;
- la figure 7A représente schématiquement une vue en perspective d'un dispositif de mesure en colonne positionné face à un porteur équipé d'un clip monté sur une monture de lunettes ; la figure 7B représente le dispositif de la figure 7A avec une coupe partielle montrant le système d'éclairage et de capture d'image ;
- la figure 8A représente schématiquement une vue de trois quart arrière d'un dispositif de mesure en colonne positionné face à un porteur équipé d'un clip fixé sur une monture de lunettes dans des conditions de mesure en vision de loin ; la figure 8B représente schématiquement une vue du porteur équipé d'un clip prise depuis la caméra d'imagerie en vision de loin ; la figure 8C représente le dispositif de la figure 8A montrant le système d'éclairage et de capture d'image ;
- la figure 9 représente schématiquement une vue de côté d'un dispositif de mesure selon un premier mode de réalisation, dans des conditions de mesure en vision de loin ;
- la figure 10 représente schématiquement une vue de côté d'un dispositif de mesure selon le premier mode de réalisation, dans des conditions de mesure en vision de près ;
- la figure 11A représente schématiquement une vue d'ensemble d'un dispositif de mesure en colonne des figures 9 et 10, positionné face à un porteur équipé d'un clip monté sur une monture de lunettes dans des conditions de mesure en vision de près ; la figure 11B représente schématiquement une tablette de lecture en vision de près ; la figure 11C représente schématiquement une vue du porteur équipé d'un clip prise depuis la caméra d'imagerie en vision de près ;
- la figure 12 représente schématiquement une vue d'ensemble d'un dispositif de mesure en colonne selon un autre mode de réalisation ;
- la figure 13 représente schématiquement le fonctionnement du système optique de renvoi du dispositif de la figure 12.

Dans le présent document, on entend par « comportement visuel » un ensemble de paramètres comprenant la distance de vision, ou proximité d'une cible, la position des pupilles, l'orientation de l'axe du regard, la vergence du regard, l'abaissement du regard par rapport à une ligne horizontale, ainsi que la posture corporelle et la posture de tête du sujet.

En particulier on définit un comportement visuel en vision de loin (VL), dans lequel la cible de stimulation du regard est disposée à la hauteur des yeux, l'individu regardant droit devant lui, la tête étant droite, la cible de stimulation étant à une distance de l'individu telle qu'elle présente une proximité inférieure à deux dioptries. On définit aussi un comportement visuel en vision de près (VP), dans lequel une cible de stimulation du regard est disposée de manière à ce que la tête de l'individu soit inclinée et/ou son regard soit abaissé d'un angle compris entre - 10 et -50 degrés par rapport à une ligne horizontale, la cible étant à une distance telle qu'elle a une proximité comprise entre 1 et 5 dioptries.

### Dispositif

Sur la figure 3, on a représenté un exemple d'interface graphique d'un dispositif de mesure combiné. Dans cet exemple, un sujet porte une monture de lunettes 1 sur laquelle est fixé un clip 2 muni de marqueurs 21, 25, 26. Il existe différents types de marqueurs : des marqueurs en noir et blanc et des marqueurs en niveaux de gris pour permettre d'ajuster l'éclairement, notamment dans le visible. Le dispositif de mesure acquiert une image, ou une séquence d'images, de l'individu portant la monture et en déduit une mesure combinée des paramètres de réfraction oculaire des deux yeux et d'au moins un paramètre géométrico-morphologique (par exemple un paramètre de monturisation) dans un comportement visuel déterminé et identique pour les deux types de mesure. Pour cela, l'appareil utilise un dispositif combinant un système d'éclairage nécessaire à la photoréfraction et un détecteur d'image permettant de mesurer la réfraction des deux yeux ainsi que les paramètres de monturisation. Le détecteur d'image collecte non seulement une image du visage du porteur mais aussi un signal par réfraction du faisceau d'éclairage sur les yeux du porteur.

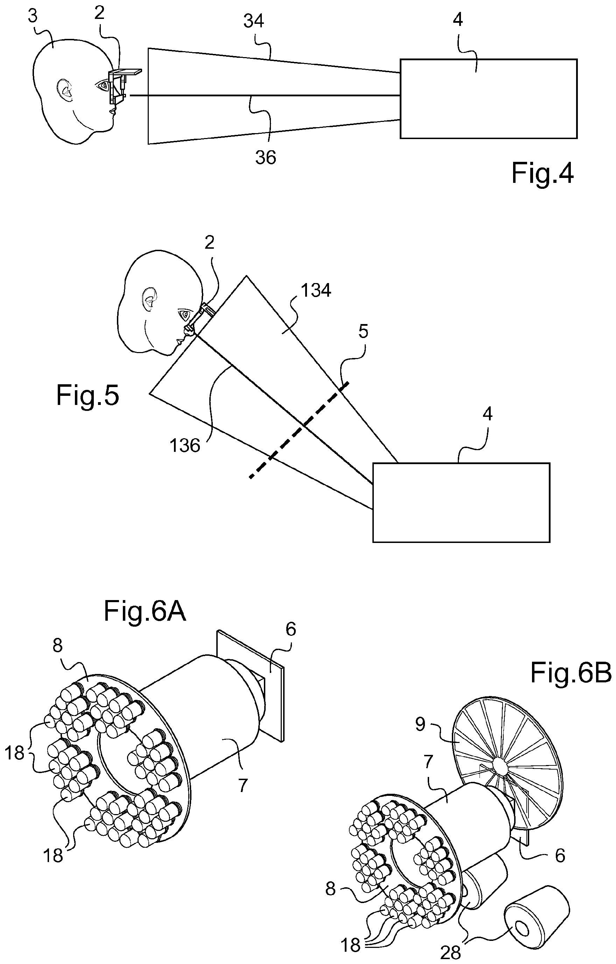

La figure 4 est une vue schématique d'un dispositif de mesure selon un premier mode de réalisation. Le sujet 3 porte une monture de lunettes équipée d'un clip 2 portant des marqueurs tels que par exemple représentés sur la figure 3. Le dispositif de mesure 4 comporte une cible de stimulation du regard de manière à stimuler le regard du sujet dans un comportement visuel déterminé. Dans l'exemple représenté sur la figure 4, le comportement visuel correspond à la vision de loin, la cible ayant une valeur de proximité P1 inférieure à deux dioptries, l'axe du regard est horizontal et la vision est binoculaire. Le dispositif de mesure 4 comporte aussi un système d'éclairage pour générer un faisceau d'éclairage 34. De façon avantageuse, le système d'éclairage comporte au moins une source infrarouge pour générer un faisceau d'éclairage infrarouge de manière à éclairer les yeux du sujet sans l'éblouir. Le faisceau d'éclairage 34 a une divergence suffisante pour éclairer aussi au moins une partie du visage du sujet autour des yeux. Par exemple, le système d'éclairage comporte un ensemble de diodes électro-luminescentes (LED) infrarouge. De façon avantageuse, le clip 2 comporte des marqueurs rétro-réflecteurs fonctionnant dans le domaine infrarouge. Le dispositif de mesure 4 comporte aussi un système de capture d'image apte à former une image d'au moins une partie du visage du sujet 3 portant la monture 1. Le système de capture d'image comporte avantageusement un système optique apte à former une image du visage du sujet dans le plan d'un capteur d'image. Avantageusement, le faisceau d'éclairage 34 est centré sur l'axe optique 36 du système de capture d'image. De façon préférée, le système optique de capture d'image est apte à former une image infrarouge et le système de capture d'image comporte une caméra infrarouge. Par réfraction sur les milieux oculaires de l'oeil, le faisceau d'éclairage 34 forme un signal de réfraction oculaire. Par réflexion et/ou diffusion sur le visage et sur les marqueurs rétro-réflecteurs, le faisceau d'éclairage 34 forme aussi un faisceau de rétro-réflexion et/ou rétro-diffusion en direction du système de capture d'image. Le système de capture d'image reçoit une image du visage du porteur, de la monture et des marqueurs rétro-réflecteurs du clip. Le sujet étant toujours dans la même posture, le système de capture d'image reçoit aussi un signal représentatif du faisceau de réfraction oculaire sur les yeux du sujet. Un calculateur permet d'extraire de l'image détectée au moins un paramètre de réfraction oculaire et au moins un paramètre de monturisation. L'image du sujet étant prise dans un comportement visuel déterminé, les mesures de réfraction oculaire et de paramètres de monturisation sont ainsi réalisées pour un même comportement visuel du sujet (dans l'exemple représenté : tête droite, regard horizontal, valeur de proximité P1 de la cible inférieure à deux dioptries).

La figure 5 est une vue schématique d'un dispositif de mesure dans une configuration de mesure pour un autre comportement visuel du sujet, par exemple un comportement en vision de près. Le dispositif de mesure est analogue à celui décrit en lien avec la figure 4, le sujet 3 portant une monture 1 équipée d'un clip 2. Le dispositif de la figure 5 comporte en outre, pour la VP, une cible 5 ayant une valeur de proximité P2 comprise entre deux et cinq dioptries et disposée de manière à stimuler un comportement en vision de près, avec un axe du regard incliné d'un angle compris entre -10 et -50 degrés par rapport à une ligne horizontale, en vision est binoculaire. La cible 5 peut se présenter sous la forme d'une tablette. Le système d'éclairage génère un faisceau d'éclairage 134 dirigé vers les yeux et au moins une partie du visage du sujet de manière à éclairer simultanément les deux yeux, la monture et le clip. Le faisceau d'éclairage 134 est dirigé directement vers les yeux du sujet ou par un système optique de renvoi suivant l'axe optique incliné 136. Le système de capture d'image reçoit une image ou une séquence d'images du visage du porteur et aussi un signal représentatif du faisceau de réfraction oculaire sur les yeux du sujet pour le comportement visuel en VP. De manière similaire aux mesures en VL, un calculateur permet d'extraire de l'image détectée au moins un paramètre de réfraction oculaire et au moins un paramètre de monturisation pour le comportement visuel en VP (dans l'exemple représenté : tête et/ou regard inclinés par rapport à l'horizontale, valeur de proximité P2 de la cible comprise entre deux et cinq dioptries).

A partir des mesures en VL et en VP, le calculateur peut en déduire une mesure différenciée des paramètres de réfraction entre la VL et la VP. Par exemple, on peut ainsi obtenir une mesure d'astigmatisme différenciée entre la VL et la VP.

La figure 6A représente schématiquement un détail du système de mesure 4 comprenant un système d'éclairage 8 constitué par des LEDs infrarouge 18 disposées sur un anneau en cercles concentriques autour de l'axe du système de capture d'image. Les faisceaux image de retour passent par l'ouverture centrale de l'anneau. Le système de capture d'image comporte un objectif 7, de préférence fonctionnant au moins dans l'infrarouge, et une caméra 6, de préférence infrarouge aussi. Dans l'exemple représenté, les LEDs 18 sont disposées en secteurs annulaires autour de l'axe de l'objectif 7 de la caméra 6. Les LEDs 18 sont allumées suivant une séquence prédéterminée de manière à former un signal permettant d'effectuer une détermination de la réfraction oculaire.

Préférentiellement, la caméra 6 enregistre une séquence d'images de manière synchrone avec une séquence d'allumage de différentes LEDs 18.

Préférentiellement, les Leds 18 sont disposées suivant six secteurs (au moins trois secteurs), chaque secteur étant piloté indépendamment de manière à mesurer la réfraction dans la méridienne des deux yeux correspondant à ce secteur. La mesure des différentes valeurs de réfraction pour chaque méridienne permet alors de déterminer la réfraction complète de l'oeil (la sphère, le cylindre, l'axe de cylindre).

Un calculateur permet, à partir de la séquence d'images enregistrées, de déterminer une mesure de réfraction oculaire objective.

Pour la mesure des paramètres de monturisation, on allume préférentiellement l'ensemble des secteurs afin d'éclairer de manière homogène le visage et afin d'augmenter la quantité de lumière reçue.

Une séquence d'allumage préférentielle consiste à allumer l'ensemble des secteurs lors de la mise en position du porteur, ceci afin de visualiser au mieux son visage, de réaliser une première prise d'image, puis d'allumer chaque secteur indépendamment et de prendre une image pour chaque secteur.

La séquence complète ou l'allumage indépendant des différents secteurs peuvent être réalisés de multiples fois, afin de mesurer la réfraction en continu, ou de réaliser un moyennage des valeurs obtenues sur les paramètres de réfraction ou sur les paramètres de monturisation.

Alternativement, on peut aussi envisager de n'utiliser qu'un seul secteur pour l'ensemble des mesures, et dans ce cas on réalise une mesure de réfraction limitée (mesure de puissance réfractive dans l'axe du segment). Dans ce cas, la séquence n'est constituée que d'une seule image et on déduit de cette image à la fois les paramètres de monturisation et de réfraction (mode dégradé).

La figure 6B représente un système de mesure 4 selon une variante comportant en outre un dispositif d'éclairage visible 28. Avantageusement, le système d'éclairage à LEDs infrarouge 18 est mis en oeuvre pour des mesures de photoréfraction et le système d'éclairage visible 28 est activé pour générer un éclairage dans le visible pour les mesures de monturisation et pour stimuler l'oeil directeur.

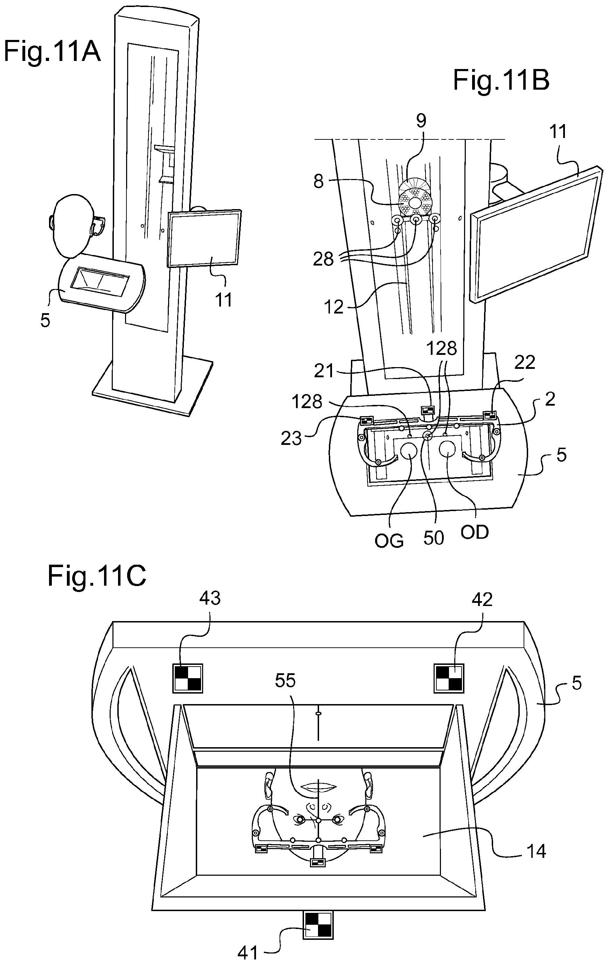

Une difficulté de la mesure sans contrainte est le positionnement du porteur en face de la caméra pour la mesure en VP. En effet dans le cas d'un stimulus en VP, le porteur à tendance à positionner la cible de VP (par exemple une tablette 5) vers son oeil directeur avec le risque de sortir du champ de la caméra. Pour cela, on dispose de trois LEDs visibles 28. La LED centrale s'allume et on demande au porteur de regarder l'image 128 de la LED 28 centrale à travers un cercle 50 de la tablette 5 (figure 11B). Le calculateur en déduit l'oeil directeur. Puis en fonction de l'oeil directeur, on allume une des deux Leds 28 extérieures pour forcer le porteur à se repositionner en face du système de mesure.

Les sources d'éclairage visible 28 et infrarouge 8 peuvent être allumées simultanément ou séquentiellement. Sur la figure 6B, le système de capture d'image comporte en outre une roue à filtre 9 disposée entre l'objectif 7 et le capteur infrarouge 6. La roue à filtre 9 comporte différents filtres tels que par exemple un filtre infrarouge, des filtres rouge-vert-bleu (RGB) de manière à sélectionner une image spectrale dans le visible ou l'infrarouge respectivement.

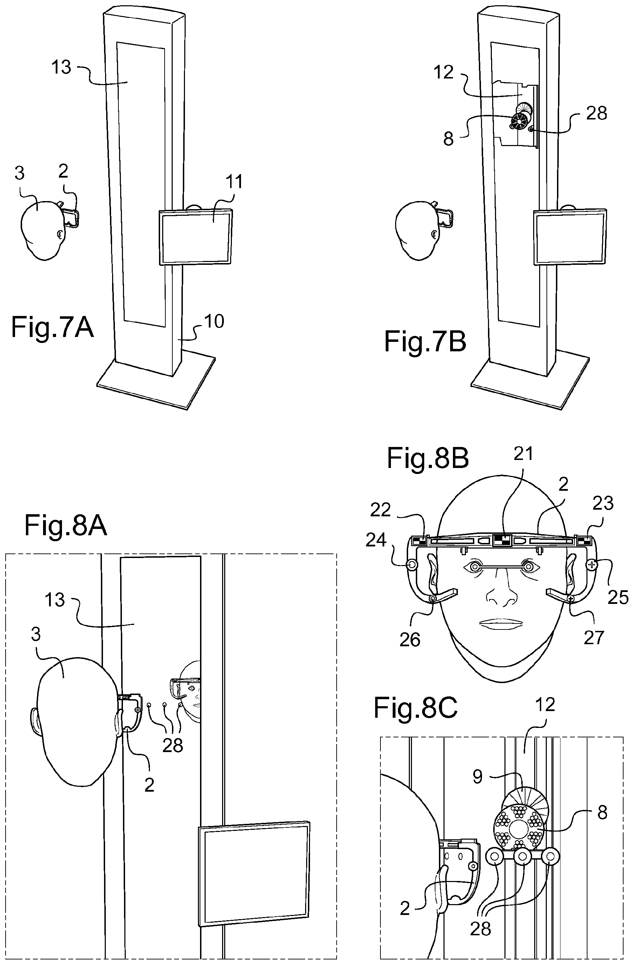

Sur les figures 7 à 8, on a représenté un premier mode de réalisation d'un système de photoréfraction intégré dans une colonne de prise de mesure type « Visioffice ». La colonne 10 supporte tous les éléments du dispositif de mesure, mis à part le clip 2 fixé sur la monture. La colonne 10 supporte en particulier un écran de visualisation 11 qui permet à la fois de contrôler l'alignement de la tête de mesure et d'afficher les résultats. L'écran de visualisation 11 est relié à un calculateur qui collecte l'ensemble des données et traite ces données. De façon avantageuse, le calculateur est intégré dans la colonne 10. La colonne 10 comporte une lame séparatrice 13 au moins partiellement réfléchissante dans le visible et transparente dans l'infrarouge. La lame séparatrice 13 peut aussi être partiellement transparente dans le visible. La lame séparatrice 13 fait office de miroir et masque au regard le système d'éclairage ainsi que le système de capture d'image. Les figures 7B et 8C montrent en coupe partielle la position du système d'éclairage et du système de capture d'image alignés face à un porteur dans une posture en VL.

Sur les figures 7B et 8C on observe que la colonne 10 comporte un rail de guidage 12. Avantageusement, le système d'éclairage et de capture d'image est monté sur un chariot de translation mobile le long du rail de guidage. De manière préférée, le déplacement du chariot est motorisé. On déplace le chariot de manière à aligner sensiblement l'axe de la caméra sur l'axe du regard du porteur en vision de loin. Pendant ce réglage, la caméra acquiert des images. L'écran de visualisation 11 permet de contrôler la position de la caméra par rapport au visage du porteur. La colonne est construite de manière à ce que l'axe de la caméra soit perpendiculaire à la surface du miroir 13. Pour simuler une vision au loin, le porteur peut par exemple regarder son reflet 33 dans le miroir semi-transparent 13 (cf Figure 8A). Le porteur est alors placé face à l'axe de la caméra. On ajuste la hauteur du système de capture d'image de façon à s'adapter à la taille des porteurs lors d'une mesure debout en VL. Le porteur est équipé de sa monture de lunettes sur laquelle est ajouté un clip 2 muni de marqueurs 21, 22, 23, 24, 25, 26, 27 permettant de localiser la monture de lunettes dans l'espace et par rapport aux pupilles (cf Figures 8B-8C).

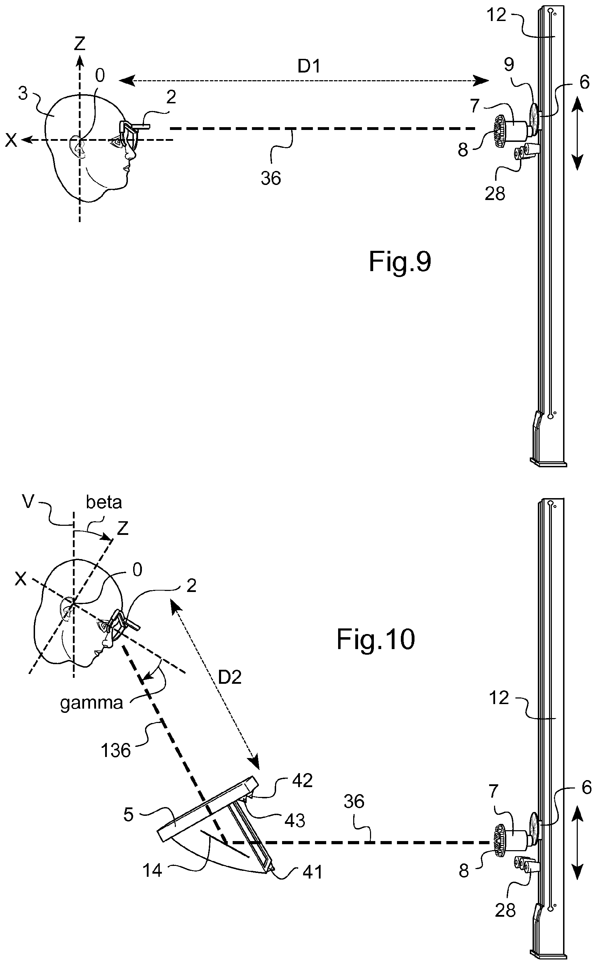

La figure 9 représente schématiquement la configuration pour une mesure en VL d'un dispositif en colonne tel que celui représenté sur les figures 7 et 8. Une caméra 6 est munie d'un objectif 7 et d'une roue à filtre 9. Un système d'éclairage comporte d'une part des LEDs infrarouge disposées autour de l'axe de la caméra 6 et d'autre part un système d'éclairage visible 28 disposé juste en dessous de la caméra 6. Comme décrit en lien avec la figure 6B, le système d'éclairage visible 28 permet de gérer l'oeil directeur du porteur. L'individu porte une monture de lunettes 1 équipée d'un clip 2. Sur la figure 9, l'individu est en posture de loin, l'axe de son regard étant horizontal. On définit un repère (OX, OZ) lié à la tête de l'individu. L'axe X passe par le centre de rotation (CRO) de l'oeil et par le porion O du porteur, le porion étant le point du crâne le plus élevé du conduit auditif, qui correspond au tragion de l'oreille, c'est-à-dire le point le plus élevé du tragus de l'oreille. L'axe Z passe par le point O et est perpendiculaire à l'axe X. En vision de loin, la tête est droite (l'axe X est horizontal et l'axe Z vertical) et le regard est droit devant, l'axe du regard étant parallèle à l'axe X horizontal. L'axe 36 du système optique de capture d'image est aussi horizontal. Le dispositif comporte des moyens de mesure de la distance D1 entre le visage de l'individu et le système de capture d'image. Par exemple, on peut utiliser les marqueurs du clip 2 pour mesurer la distance D1. On contrôle que la distance D1 est supérieure à une distance minimum, de manière à ce que la proximité de la cible de vision de loin (par exemple le reflet 33 dans le miroir) soit inférieure à deux dioptries.

De plus, la mesure de la distance D1 permet de déterminer le facteur d'échelle (taille d'un pixel dans l'espace objet) et ainsi de mesurer précisément les paramètres de monturisation (DVO,CRO,PD,H). Cette distance D1 permet aussi de s'assurer que l'individu est positionné à une distance compatible avec des mesures de qualité, par exemple qu'il est dans le plan de mise au point du système d'acquisition d'image. Enfin, cette distance est utilisée afin d'améliorer la précision de la mesure de la réfraction, en particulier pour les réfractions fortes.

On peut ainsi comparer expérimentalement une loi préétablie de variation de la réfraction par rapport à la réfraction mesurée en fonction de la distance et connaissant la distance pour l'individu et sa réfraction mesurée, on peut ainsi corriger l'erreur de distance sur la réfraction.

La figure 10 représente schématiquement la configuration pour une mesure en VP d'un dispositif en colonne. Dans la posture en VP, l'axe Z est incliné par rapport à un axe vertical V d'un angle beta, aussi appelé angle de tête. L'axe du regard est incliné par rapport à l'axe X d'un angle gamma, aussi appelé angle d'abaissement du regard. Sur la figure 10, l'individu est en posture de près, l'axe de son regard étant abaissé d'un angle d'environ 60° (composé par exemple d'un angle de tête beta de 25 degrés par rapport à la verticale et d'un angle d'abaissement du regard gamma de 35 degrés par rapport à la tête). Dans le cas d'une vision rapprochée, on peut utiliser une tablette 5 sur laquelle sont inscrits des motifs formant une cible en vision de près. La distance D2 détermine la proximité de la cible de vision de près. La tablette 5 permet aussi de mesurer le comportement visuel du porteur (abaissement du regard, posture de la tête, distance de lecture...). Pour cela la tablette 5 est équipée d'un miroir de renvoi 14 disposé entre la tablette 5 et le système d'éclairage et de capture d'image. Le chariot de déplacement permet de modifier la hauteur de la tête de mesure. L'axe 36 de la caméra reste horizontal. Le miroir plan de renvoi 14 permet de dévier l'axe 36 de la caméra et du système d'éclairage sur un axe 136 aligné avec l'axe du regard de l'individu en vision de près (Fig. 10).

Le réglage de l'orientation de la tablette 5 est réalisé par l'individu. Par exemple, le chariot de déplacement positionne l'axe de la caméra/système d'éclairage à une hauteur inférieure, correspondant à la hauteur de la tablette lorsque l'individu est en position de lecture. L'individu oriente alors la tablette de manière à percevoir (la tablette est transparente en périphérie par exemple) l'axe caméra/système au centre de la tablette, puis observe sans bouger d'orientation un motif sérigraphié 50 sur la tablette 5, représenté par exemple sur la figure 11B.

La figure 11A représente une vue d'ensemble du dispositif de mesure en colonne muni d'une tablette 5 pour la mesure en VP. La figure 11B représente plus en détail le système d'éclairage visible 28, l'écran de visualisation et de contrôle, la tablette 5 ainsi que les positions du clip 2, de l'oeil droit OD et de l'oeil gauche OG du porteur. La figure 11C représente une image prise par la caméra face au miroir de renvoi 14. On observe une image du visage de l'individu portant une monture et un clip. Un cadre de guidage permet au sujet d'affiner son positionnement latéralement (par exemple relativement à l'axe central 55 sérigraphié sur le miroir 14) et/ou en distance (D2) par rapport au dispositif de mesure. Les marqueurs 41, 42, 43 permettent de contrôler le centrage et l'orientation de la tablette 5 par rapport à l'axe 36 du système de capture d'image.

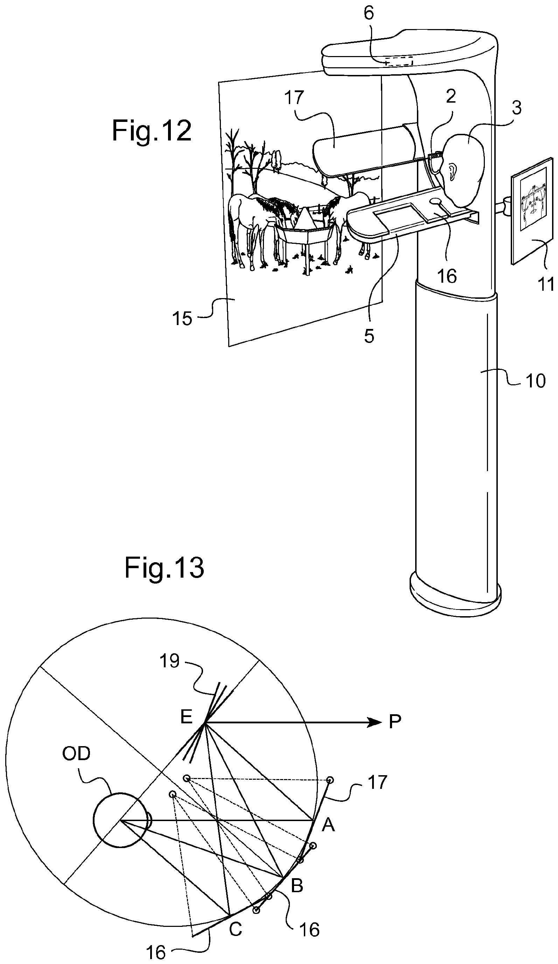

Sur la figure 12, on a représenté un autre mode de réalisation d'un dispositif de mesure en colonne. Le dispositif comporte une colonne 10 qui intègre un système de mesure, un calculateur et une interface graphique 11 de contrôle et d'affichage de résultats de mesure. De façon avantageuse, le système de mesure comprenant la caméra 6 et le système d'éclairage est situé dans la partie supérieure de la colonne. Le système de mesure est orienté vers le bas, en direction d'une cible de vision de près 5. Avantageusement, selon ce mode de réalisation, le système de mesure est fixe en hauteur et orientable, par exemple via un petit miroir de renvoi, quel que soit le comportement visuel et quelle que soit la taille de l'individu. Le dispositif de la figure 12 comporte en outre une lame séparatrice mobile entre au moins une position 16 correspondant à un comportement visuel en VP et une autre position 17 correspondant à un comportement visuel en VL. Dans cette figure, la lame 17 du haut correspondant à la vision de loin est fixe et la lame 16 du bas a la possibilité de se déplacer angulairement pour gérer plusieurs angles d'abaissement du regard. On a représenté schématiquement la position d'un oeil OD du sujet lors des mesures. Le sujet porte une monture équipée d'un clip. La lame séparatrice est placée dans une première position de mesure 17 en VL. Avantageusement, la hauteur de la colonne est ajustable en fonction de la taille du sujet. Dans un premier comportement visuel (VL), on demande au sujet d'observer un point particulier sur une cible 15 ayant une proximité inférieure à deux dioptries. Préférentiellement, la lame séparatrice 17 est transparente dans le domaine visible et réfléchissante dans le domaine infrarouge. Le sujet peut donc observer la cible 15 en VL en transparence à travers la lame séparatrice en position 17. La lame séparatrice 17 est orientée de manière à ce que le système de capture d'image 6, situé dans la partie supérieure, soit apte à réaliser une acquisition d'image du porteur (voir figure 12). Pour la mesure en VP, on demande au sujet d'observer une autre cible 5 située derrière la lame séparatrice 16. La cible 5 a une proximité comprise entre deux et cinq dioptries. Un miroir orientable permet d'aligner l'axe optique du système de capture d'image sur l'axe de visée du sujet. La lame séparatrice 16 est orientable angulairement, conjointement avec la cible 5 de manière à simuler différents angles d'abaissement du regard en vision de près. Le système de capture d'image suit les mouvements de la lame séparatrice et de la cible afin de maintenir l'alignement entre l'axe optique du système de capture d'image et l'axe de visée du sujet.

Sur la figure 13 on a représenté un exemple de dispositif optique permettant de diriger le faisceau image vers une caméra fixe. On a représenté schématiquement en vue de côté, la position A fixe de la lame séparatrice 17 et deux positions distinctes B et C de la lame séparatrice 16. Un miroir orientable 19 permet de renvoyer l'axe optique de mesure dans une direction constante définie par la droite EP selon qu'on utilise la lame séparatrice 17 en position A ou respectivement de la lame séparatrice 16 en position B ou C. L'axe optique de mesure est ainsi aligné sur l'axe optique de visée, quel que soit l'angle d'abaissement du regard.

De manière préférée, le dispositif de l'invention permet de stimuler différents comportements de vision de l'individu portant des lunettes par exemple en utilisant plusieurs cibles de stimulation du regard ou en modifiant la position et la valeur de proximité d'une cible, le porteur regardant chaque cible dans une position naturelle. Conjointement, un système de capture d'image permet d'effectuer des mesures dans plusieurs conditions de vision, telles qu'un comportement en vision de loin et/ou un comportement en vision de près. Pour cela, l'appareil utilise un système d'imagerie combinant l'éclairage nécessaire à la photoréfraction et la prise d'image permettant de mesurer la réfraction des deux yeux ainsi que les paramètres de monturisation.

Les avantages d'une telle solution sont :
- la combinaison (simultanée ou séquentielle) de deux mesures pouvant être longues et généralement effectuées à des endroits différents. Le temps de mesure est réduit et le porteur n'a pas à se déplacer, tout est centralisé sur le même instrument. Le dispositif n'induit pas d'accommodation proximale car le champ est libre devant le sujet (il n'y a pas d'appui frontal ou mentonnière).
- La réfraction est effectuée dans le cadre d'un comportement naturel du porteur (port de tête, vision binoculaire, distance de lecture personnelle,...)
- Le dépistage de différents troubles de la vision tels que la cataracte, kératocône ou les problèmes de phorie.

## Revendications

1. Dispositif de mesure de réfraction oculaire objective et d'au moins un paramètre géométrico-morphologique d'un individu, ledit dispositif comprenant :
- au moins une première cible de stimulation du regard (15) ayant un centre disposé dans une première position de manière à stimuler le regard de l'individu dans une première posture associée à une première valeur de proximité P1 et un premier axe de visée,
- un système d'éclairage comportant au moins une source lumineuse (8, 18, 28), le système d'éclairage étant apte à générer au moins un faisceau d'éclairage en direction des yeux de l'individu dans la première posture,
- un système de capture d'image (6, 7),
- un calculateur,
**caractérisé en ce que** le dispositif de mesure comprend en outre :
- au moins une deuxième cible de stimulation du regard (5) ayant un centre disposé dans une deuxième position de manière à stimuler le regard de l'individu dans une deuxième posture associée à une deuxième valeur de proximité (P2) et un deuxième axe de visée,
- un système optique de renvoi (14, 16) disposé entre d'une part la deuxième cible de stimulation du regard (15) et d'autre part le système de capture d'image (6,7) et le système d'éclairage (8, 18, 28), le système optique de renvoi (14, 16) étant adapté, dans une orientation déterminée, pour renvoyer le faisceau d'éclairage en direction des yeux de l'individu regardant la deuxième cible (5) dans une deuxième posture,
- au moins une lame séparatrice (13, 17) disposée entre, d'une part, la première cible de stimulation du regard (15) et, d'autre part, le système de capture d'image (6, 7) et ladite au moins une lame séparatrice (13) masquant au regard le système d'éclairage et le système de capture d'image (6, 7), ou bien la lame séparatrice (17) est orientée de manière à ce que le système de capture d'images (6, 7) soit apte à réaliser une acquisition d'image du porteur, tout en permettant au sujet d'observer la cible (15) en transparence à travers la lame séparatrice, et **en ce que**
- le système de capture d'image (6, 7) est adapté pour obtenir une première acquisition d'image comprenant une partie du visage entourant les yeux de l'individu dans la première posture, et le système de capture d'image (6, 7) étant adapté pour obtenir via le système optique de renvoi (14,16) une deuxième acquisition d'image comprenant une partie du visage entourant les yeux de l'individu dans la deuxième posture, et
- le calculateur est adapté pour recevoir et extraire de ladite au moins une première acquisition d'image d'une part une première mesure d'au moins un paramètre géométrico-morphologique de l'individu dans la première posture et d'autre part une première mesure de réfraction oculaire objective par réfraction du faisceau d'éclairage sur les yeux de l'individu dans la première posture et le calculateur étant adapté pour extraire de ladite deuxième acquisition d'image d'une part une deuxième mesure d'un paramètre géométrico-morphologique de l'individu dans la deuxième posture et d'autre part une deuxième mesure de réfraction oculaire objective par réfraction du faisceau d'éclairage sur les yeux de l'individu dans la deuxième posture.

2. Dispositif de mesure selon la revendication 1, dans lequel ledit au moins un paramètre géométrico-morphologique est constitué d'au moins un paramètre de monturisation d'un individu portant une monture de lunettes (1).

3. Dispositif de mesure selon l'une des revendications 1 à 2 dans lequel le système d'éclairage (8, 18, 28) générant un faisceau d'éclairage et le système de capture d'image (6, 7) définissant un axe optique, le système d'éclairage (8, 18, 28) et le système de capture d'image (6, 7) sont disposés l'un par rapport à l'autre de manière à ce que le faisceau d'éclairage soit centré sur l'axe optique du système de capture d'image (6, 7).

4. Dispositif de mesure selon l'une des revendications précédentes comportant en outre des moyens de mesure de la distance entre ledit système de capture d'image (6, 7) et l'individu dans la première posture et/ou dans la deuxième posture, lesdits moyens de mesure de distance étant sélectionnés parmi : un télémètre, un système de traitement d'image basé sur la qualité image, un système de traitement d'image basé sur une mesure de marqueurs (21, 22, 23, 24, 25) d'un clip (2) fixé à une monture de lunettes (1), un système d'étalonnage ou un système de mesure de distance par ultrasons.

5. Dispositif de mesure selon l'une des revendications précédentes, comportant en outre un clip (2) muni de marqueurs (21, 22, 23, 24, 25) destiné à être monté sur une monture de lunettes (1) et dans lequel le système de capture d'image (6, 7) présente un champ image adapté pour détecter simultanément une image comprenant une partie du visage entourant les yeux de l'individu portant la monture de lunettes (1) et une image des marqueurs (21, 22, 23, 24, 25) du clip (2) monté sur la monture de lunettes (1).

6. Dispositif de mesure selon l'une des revendications précédentes, dans lequel le système d'éclairage (8) comporte au moins une source lumineuse infrarouge (18) et dans lequel le système de capture d'image (6, 7) comporte une caméra infrarouge.

7. Dispositif de mesure selon l'une des revendications précédentes comportant des moyens de déplacement et/ou d'orientation (12) du système de capture d'image (6, 7) aptes à aligner l'axe optique du système de capture d'image (6, 7) avec le premier axe de visée associé à la première cible et/ou respectivement à aligner l'axe optique du système de capture d'image(6, 7) avec le deuxième axe de visée oculaire associé à la deuxième cible.

8. Dispositif de mesure selon l'une des revendications précédentes comportant des moyens de déplacement et/ou d'orientation du système optique de renvoi (14, 16, 17) aptes à renvoyer l'axe optique du système de capture d'image vers le premier axe de visée oculaire associé à la première cible et respectivement vers le deuxième axe de visée oculaire associé à la deuxième cible.

9. Dispositif de mesure selon l'une des revendications précédentes comportant une colonne (10) supportant le système d'éclairage (8, 18, 28), le système de capture d'image (6, 7), le calculateur, au moins une première cible (5) et un écran de visualisation (11).

10. Procédé de mesure de réfraction oculaire objective et d'au moins un paramètre géométrico-morphologique d'un individu, ledit procédé comprenant les étapes suivantes :
- activer au moins une première cible de stimulation du regard (15) de manière à stimuler le regard de l'individu dans une première posture associée à une première valeur de proximité (P1) et un premier axe de visée,
- générer par un système d'éclairage au moins un faisceau d'éclairage à l'aide d'une source lumineuse (8, 18, 28) en direction des yeux de l'individu dans la première posture,
- obtenir par un système de capture d'image (6, 7) au moins une première acquisition d'image d'une partie du visage entourant les yeux de l'individu, tout en masquant au regard le système d'éclairage et le système de capture d'image (6, 7) avec une lame séparatrice (13), ou bien en utilisant une lame séparatrice (17) orientée de manière à ce que le système de capture d'images (6, 7) soit apte à réaliser une acquisition d'image du porteur, tout en permettant au sujet d'observer la cible (15) en transparence à travers la lame séparatrice,
- calculer à partir de ladite au moins une première acquisition d'image d'une part une première mesure d'au moins un paramètre géométrico-morphologique de l'individu dans la première posture et d'autre part une première mesure de réfraction oculaire objective de l'individu dans la première posture,
- activer au moins une deuxième cible de stimulation du regard (5) de manière à stimuler le regard de l'individu dans une deuxième posture associée à une deuxième valeur de proximité (P2) et un deuxième axe de visée,
- générer au moins un faisceau d'éclairage avec la source lumineuse en direction des yeux de l'individu dans la deuxième posture,
- obtenir au moins une deuxième acquisition d'image d'une partie du visage entourant les yeux de l'individu avec un système optique de renvoi (14, 16) disposé entre d'une part la deuxième cible de simulation du regard (5) et d'autre part le système de capture d'image (6, 7) et le système d'éclairage (8, 18, 28), le système optique de renvoi (14, 16) étant adapté, dans une orientation déterminée, pour renvoyer le faisceau d'éclairage en direction des yeux de l'individu regardant la deuxième cible (5) dans une deuxième posture,
- calculer à partir de ladite au moins une deuxième acquisition d'image d'une part une deuxième mesure d'au moins un paramètre géométrico-morphologique de l'individu dans la deuxième posture et d'autre part une deuxième mesure de réfraction oculaire objective de l'individu dans la deuxième posture.

11. Procédé de mesure selon la revendication 10 dans lequel ledit au moins un paramètre géométrico-morphologique est constitué d'au moins un paramètre de monturisation d'un individu portant une monture de lunettes (1), le paramètre de monturisation étant sélectionné parmi les écarts pupillaires, la distance verre-oeil, la position du centre de rotation de l'oeil, la hauteur des yeux relativement au bord inférieur de la monture.

12. Procédé de mesure selon l'une des revendications 10 à 11 dans lequel la première mesure d'au moins un paramètre géométrico-morphologique et la première mesure de réfraction oculaire objective de l'individu dans la première posture sont des mesures séquentielles ou simultanées.

13. Procédé de mesure selon l'une des revendications 10 à 12, comportant une étape supplémentaire de contrôle de la première posture et/ou de la deuxième posture de manière à ce que le premier axe de visée associé à la première posture et/ou respectivement le deuxième axe de visée associé à la deuxième posture, soit compris dans un cône centré sur l'axe optique du système de capture d'image, ledit cône ayant un angle au sommet inférieur ou égal à dix degrés.

14. Procédé de mesure selon l'une des revendications 10 à 13 comportant en outre une étape de mesure d'un angle d'abaissement du regard de l'individu.

15. Procédé de mesure selon l'une des revendications 10 à 14 comportant en outre une étape de mesure d'au moins un paramètre physiologique de vision de l'individu portant une monture de lunettes (1) dans la première et/ou deuxième posture, le paramètre physiologique de vision étant représentatif de strabisme, kératocône ou de cataracte ou de paramètres comportementaux de l'individu.

16. Procédé de mesure selon l'une des revendications 10 à 14 dans lequel l'individu portant une monture de lunettes (1) équipée de verres, le procédé comporte une étape supplémentaire de correction de la mesure de réfraction oculaire à partir d'au moins l'une des valeurs suivantes : la valeur de correction de puissance des verres ou la valeur du coefficient de transmission des verres.

## Patentansprüche

1. Messvorrichtung einer objektiven Okularrefraktion und mindestens eines geometrisch-morphologischen Parameters einer Person, wobei die Vorrichtung enthält:
- mindestens ein erstes Stimulationsziel des Blicks (15), das eine Mitte in einer ersten Position angeordnet hat, um den Blick der Person in einer ersten Körperhaltung zu stimulieren, die einem ersten Nahbereichswert P1 und einer ersten Zielachse zugeordnet ist,
- ein Beleuchtungssystem, das mindestens eine Lichtquelle (8, 18, 28) aufweist, wobei das Beleuchtungssystem mindestens einen Beleuchtungsstrahl in Richtung der Augen der Person in der ersten Körperhaltung erzeugen kann,
- ein Bilderfassungssystem (6, 7),
- einen Rechner,
**dadurch gekennzeichnet, dass** die Messvorrichtung außerdem aufweist:
- mindestens ein zweites Stimulationsziel des Blicks (5), das eine Mitte in einer zweiten Position angeordnet hat, um den Blick der Person in einer zweiten Körperhaltung zu stimulieren, die einem zweiten Nahbereichswert (P2) und einer zweiten Zielachse zugeordnet ist,
- ein optisches Umlenksystem (14, 16), das zwischen einerseits dem zweiten Stimulationsziel des Blicks (5) und andererseits dem Bilderfassungssystem (6, 7) und dem Beleuchtungssystem (8, 18, 28) angeordnet ist, wobei das optische Umlenksystem (14, 16) in einer bestimmten Ausrichtung geeignet ist, den Beleuchtungsstrahl in Richtung der Augen der Person umzulenken, die das zweite Ziel (5) in einer zweiten Körperhaltung betrachtet,
- mindestens eine Trennlamelle (13, 17), die zwischen einerseits dem ersten Stimulationsziel des Blicks (15) und andererseits dem Bilderfassungssystem (6, 7) angeordnet ist, und wobei die mindestens eine Trennlamelle (13) das Beleuchtungssystem und das Bilderfassungssystem (6, 7) vor dem Blick verbirgt, oder aber die Trennlamelle (17) so ausgerichtet ist, dass das Bilderfassungssystems (6, 7) in der Lage ist, eine Bilderfassung des Trägers durchzuführen, während es der Versuchsperson ermöglicht wird, das Ziel (15) in Transparenz durch die Trennlamelle hindurch zu beobachten, und dass
- das Bilderfassungssystem (6, 7) geeignet ist, eine erste Bilderfassung zu erhalten, die einen die Augen umgebenden Teil des Gesichts der Person in der ersten Körperhaltung enthält, und das Bilderfassungssystem (6, 7) geeignet ist, über das optische Umlenksystem (14, 16) eine zweite Bilderfassung zu erhalten, die einen die Augen umgebenden Teil des Gesichts der Person in der zweiten Körperhaltung enthält, und
- der Rechner geeignet ist, aus der mindestens einen ersten Bilderfassung einerseits einen ersten Messwert mindestens eines geometrisch-morphologischen Parameters der Person in der ersten Körperhaltung und andererseits einen ersten Messwert der objektiven Okularrefraktion durch Refraktion des Beleuchtungsstrahls auf die Augen der Person in der ersten Körperhaltung zu empfangen und zu entnehmen, und der Rechner geeignet ist, aus der zweiten Bilderfassung einerseits einen zweiten Messwert eines geometrisch-morphologischen Parameters der Person in der zweiten Körperhaltung und andererseits einen zweiten Messwert der objektiven Okularrefraktion durch Refraktion des Beleuchtungsstrahls auf die Augen der Person in der zweiten Körperhaltung zu entnehmen.

2. Messvorrichtung nach Anspruch 1, wobei der mindestens eine geometrisch-morphologische Parameter aus mindestens einem Parameter der Gestellanpassung einer ein Brillengestell (1) tragenden Person besteht.

3. Messvorrichtung nach einem der Ansprüche 1 bis 2, wobei, wenn das Beleuchtungssystem (8, 18, 28) einen Beleuchtungsstrahl erzeugt und das Bilderfassungssystem (6, 7) eine optische Achse definiert, das Beleuchtungssystem (8, 18, 28) und das Bilderfassungssystem (6, 7) so zueinander angeordnet sind, dass der Beleuchtungsstrahl auf die optische Achse des Bilderfassungssystems (6, 7) zentriert ist.

4. Messvorrichtung nach einem der vorhergehenden Ansprüche, die außerdem Messeinrichtungen des Abstands zwischen dem Bilderfassungssystem (6, 7) und der Person in der ersten Körperhaltung und/oder in der zweiten Körperhaltung aufweist, wobei die Abstandsmesseinrichtungen ausgewählt werden aus: einem Entfernungsmesser, einem Bildverarbeitungssystem basierend auf der Bildqualität, einem Bildverarbeitungssystem basierend auf einer Messung von Markierungen (21, 22, 23, 24, 25) eines an einem Brillengestell (1) befestigten Clips (2), einem Eichsystem oder einem Abstandsmesssystem durch Ultraschall.

5. Messvorrichtung nach einem der vorhergehenden Ansprüche, das außerdem einen mit Markierungen (21, 22, 23, 24, 25) versehenen Clip (2) aufweist, der dazu bestimmt ist, auf ein Brillengestell (1) montiert zu werden, und wobei das Bilderfassungssystem (6, 7) ein Bildfeld aufweist, das geeignet ist, gleichzeitig ein einen die Augen umgebenden Teil des Gesichts der das Brillengestell (1) tragenden Person enthaltendes Bild und ein Bild der Markierungen (21, 22, 23, 24, 25) des auf das Brillengestell (1) montierten Clips (2) zu erfassen.

6. Messvorrichtung nach einem der vorhergehenden Ansprüche, wobei das Beleuchtungssystem (8) mindestens eine Infrarotlichtquelle (18) aufweist, und wobei das Bilderfassungssystem (6, 7) eine Infrarotkamera aufweist.

7. Messvorrichtung nach einem der vorhergehenden Ansprüche, die Verschiebe- und/oder Ausrichteinrichtungen (12) des Bilderfassungssystems (6, 7) aufweist, die die optische Achse des Bilderfassungssystems (6, 7) mit der dem ersten Ziel zugeordneten ersten Zielachse fluchtend ausrichten und/oder die optische Achse des Bilderfassungssystems (6, 7) mit der dem zweiten Ziel zugeordneten okularen Zielachse fluchtend ausrichten können.

8. Messvorrichtung nach einem der vorhergehenden Ansprüche, die Verschiebe- und/oder Ausrichteinrichtungen des optischen Umlenksystems (14, 16, 17) aufweist, die die optische Achse des Bilderfassungssystems zur dem ersten Ziel zugeordneten ersten okularen Zielachse bzw. zur dem zweiten Ziel zugeordneten zweiten okularen Zielachse umlenken können.

9. Messvorrichtung nach einem der vorhergehenden Ansprüche, die eine Säule (10) aufweist, die das Beleuchtungssystem (8, 18, 28), das Bilderfassungssystem (6, 7), den Rechner, mindestens ein erstes Ziel (5) und einen Anzeigebildschirm (11) trägt.

10. Messverfahren einer objektiven Okularrefraktion und mindestens eines geometrisch-morphologischen Parameters einer Person, wobei das Verfahren die folgenden Schritte enthält:
- Aktivieren mindestens eines ersten Stimulationsziels des Blicks (15), um den Blick der Person in einer ersten Körperhaltung zu stimulieren, die einem ersten Nahbereichswert (P1) und einer ersten Zielachse zugeordnet ist,
- Erzeugen durch ein Beleuchtungssystem mindestens eines Beleuchtungsstrahls mit Hilfe einer Lichtquelle (8, 18, 28) in Richtung der Augen der Person in der ersten Körperhaltung,
- Erhalt durch ein Bilderfassungssystem (6, 7) mindestens einer ersten Bilderfassung eines die Augen umgebenden Teils des Gesichts der Person, wobei das Beleuchtungssystem und das Bilderfassungssystem (6, 7) mit einer Trennlamelle (13) für den Blick verdeckt werden, oder unter Verwendung einer Trennlamelle (17), die so ausgerichtet ist, dass das Bilderfassungssystems (6, 7) eine Bilderfassung des Trägers durchführen kann, wobei es der Versuchsperson ermöglicht wird, das Ziel (15) in Transparenz durch die Trennlamelle hindurch zu beobachten,
- Berechnen ausgehend von der mindestens einen ersten Bilderfassung einerseits eines ersten Messwerts mindestens eines geometrisch-morphologischen Parameters der Person in der ersten Körperhaltung und andererseits eines ersten Messwerts der objektiven Okularrefraktion der Person in der ersten Körperhaltung,
- Aktivieren mindestens eines zweiten Stimulationsziels des Blicks (5), um den Blick der Person in einer einem zweiten Nahbereichswert (P2) und einer zweiten Zielachse zugeordneten zweiten Körperhaltung zu stimulieren,
- Erzeugen mindestens eines Beleuchtungsstrahls mit der Lichtquelle in Richtung der Augen der Person in der zweiten Körperhaltung,
- Erhalt mindestens einer zweiten Bilderfassung eines die Augen umgebenden Teils des Gesichts der Person mit einem optischen Umlenksystem (14, 16), das zwischen einerseits dem zweiten Stimulationsziel des Blicks (5) und andererseits dem Bilderfassungssystem (6, 7) und dem Beleuchtungssystem (8, 18, 28) angeordnet ist, wobei das optische Umlenksystem (14, 16) in einer bestimmten Ausrichtung geeignet ist, den Beleuchtungsstrahl in Richtung der Augen der Person umzulenken, die das zweite Ziel (5) in einer zweiten Körperhaltung betrachtet,
- Berechnen ausgehend von der mindestens einen zweiten Bilderfassung einerseits eines zweiten Messwerts mindestens eines geometrisch-morphologischen Parameters der Person in der zweiten Körperhaltung und andererseits eines zweiten Messwerts der objektiven Okularrefraktion der Person in der zweiten Körperhaltung.

11. Messverfahren nach Anspruch 10, wobei der mindestens eine geometrisch-morphologische Parameter aus mindestens einem Parameter der Gestellanpassung einer ein Brillengestell (1) tragenden Person besteht, wobei der Gestellanpassungsparameter ausgewählt wird aus den Pupillenabständen, dem Glas-Auge-Abstand, der Position des Drehzentrums des Auges, der Höhe der Augen bezüglich des unteren Rands des Gestells.

12. Messverfahren nach einem der Ansprüche 10 bis 11, wobei der erste Messwert des mindestens einen geometrisch-morphologischen Parameters und der erste Messwert der objektiven Okularrefraktion der Person in der ersten Körperhaltung sequentielle oder gleichzeitige Messwerte sind.

13. Messverfahren nach einem der Ansprüche 10 bis 12, das einen zusätzlichen Schritt der Kontrolle der ersten Körperhaltung und/oder der zweiten Körperhaltung aufweist, damit die der ersten Körperhaltung zugeordnete erste Zielachse und/oder die der zweiten Körperhaltung zugeordnete zweite Zielachse in einem Kegel enthalten ist, der auf die optische Achse des Bilderfassungssystems zentriert ist, wobei der Kegel einen Spitzenwinkel kleiner als oder gleich zehn Grad hat.

14. Messverfahren nach einem der Ansprüche 10 bis 13, das außerdem einen Schritt des Messens eines Senkwinkels des Blicks der Person aufweist.

15. Messverfahren nach einem der Ansprüche 10 bis 14, das außerdem einen Schritt des Messens mindestens eines physiologischen Sehparameters der ein Brillengestell (1) tragenden Person in der ersten und/oder zweiten Körperhaltung aufweist, wobei der physiologische Sehparameter für Schielen, Hornhautkegel oder grauen Star oder Verhaltensparameter der Person repräsentativ ist.

16. Messverfahren nach einem der Ansprüche 10 bis 14, wobei, wenn die Person ein mit Gläsern ausgestattetes Brillengestell (1) trägt, das Verfahren einen zusätzlichen Schritt der Korrektur des Okularrefraktionsmesswerts ausgehend von mindestens einem der folgenden Werte aufweist: der Korrekturwert der Stärke der Gläser oder der Wert des Übertragungskoeffizienten der Gläser.

## Claims

1. Device for measuring objective ocular refraction and at least one geometrico-morphological parameter of an individual, said device comprising:
- at least one first target for stimulating the gaze (15) having a center disposed in a first position so as to stimulate the gaze of the individual in a first posture associated with a first proximity value P1 and a first sighting axis,
- a lighting system comprising at least one luminous source (8, 18, 28), the lighting system being able to generate at least one lighting beam directed toward the eyes of the individual in the first posture,
- an image capture system (6, 7),
- a computer,
**characterized in that** the measuring device furthermore comprises:
- at least one second target for stimulating the gaze (5) having a center disposed in a second position so as to stimulate the gaze of the individual in a second posture associated with a second proximity value (P2) and a second sighting axis,
- an optical return system (14, 16) disposed between on the one hand the second target for stimulating the gaze (15) and on the other hand the image capture system (6, 7) and the lighting system (8, 18, 28), the optical return system (14, 16) being adapted, in a determined orientation, to return the lighting beam toward the eyes of the individual looking at the second target (5) in a second posture,
- at least one splitting plate (13, 17) disposed between, on the one hand, the first target for stimulating the gaze (15) and, on the other hand, the image capture system (6, 7) and said at least one splitting plate (13) masking the lighting system and the image capture system (6, 7) from the gaze, or else the splitting plate (17) is oriented in such a way that the image capture system (6, 7) is able to carry out an image acquisition of the wearer, while allowing the subject to observe the target (15) in transparency through the splitting plate, and **in that**
- the image capture system (6, 7) is adapted for obtaining a first image acquisition comprising a part of the face surrounding the eyes of the individual in the first posture, the image capture system (6, 7) being adapted for obtaining via the optical return system (14, 16) a second image acquisition comprising a part of the face surrounding the eyes of the individual in the second posture, and
- the computer is adapted for receiving and extracting from said at least one first image acquisition on the one hand a first measurement of at least one geometrico-morphological parameter of the individual in the first posture and on the other hand a first measurement of objective ocular refraction by refraction of the lighting beam on the eyes of the individual in the first posture and the computer being adapted for extracting from said second image acquisition on the one hand a second measurement of a geometrico-morphological parameter of the individual in the second posture and on the other hand a second measurement of objective ocular refraction by refraction of the lighting beam on the eyes of the individual in the second posture.

2. Measuring device according to Claim 1, in which said at least one geometrico-morphological parameter consists of at least one frame-fitting parameter for an individual wearing a spectacles frame (1).

3. Measuring device according to one of Claims 1 to 2, in which the lighting system (8, 18, 28) generating a lighting beam and the image capture system (6, 7) defining an optical axis, the lighting system (8, 18, 28) and the image capture system (6, 7) are disposed with respect to one another in such a way that the lighting beam is centered on the optical axis of the image capture system (6, 7).

4. Measuring device according to one of the preceding claims, furthermore comprising means for measuring the distance between said image capture system (6, 7) and the individual in the first posture and/or in the second posture, said distance measuring means being selected from among: a telemeter, an image processing system based on the image quality, an image processing system based on a measurement of markers (21, 22, 23, 24, 25) of a clip (2) fixed to a spectacles frame (1), a calibration system or a system for measuring distance by ultrasounds.

5. Measuring device according to one of the preceding claims, furthermore comprising a clip (2) furnished with markers (21, 22, 23, 24, 25) which is intended to be mounted on a spectacles frame (1) and in which the image capture system (6, 7) exhibits an image field adapted for simultaneously detecting an image comprising a part of the face surrounding the eyes of the individual wearing the spectacles frame (1) and an image of the markers (21, 22, 23, 24, 25) of the clip (2) mounted on the spectacles frame (1).

6. Measuring device according to one of the preceding claims, in which the lighting system (8) comprises at least one infrared luminous source (18) and in which the image capture system (6, 7) comprises an infrared camera.

7. Measuring device according to one of the preceding claims comprising means of displacement and/or orientation (12) of the image capture system (6, 7) which are able to align the optical axis of the image capture system (6, 7) with the first sighting axis associated with the first target and/or respectively to align the optical axis of the image capture system (6, 7) with the second ocular sighting axis associated with the second target.

8. Measuring device according to one of the preceding claims, comprising means of displacement and/or of orientation of the optical return system (14, 16, 17) which are able to return the optical axis of the image capture system to the first ocular sighting axis associated with the first target and respectively to the second ocular sighting axis associated with the second target.

9. Measuring device according to one of the preceding claims, comprising a column (10) supporting the lighting system (8, 18, 28), the image capture system (6, 7), the computer, at least one first target (5) and a viewing screen (11).

10. Method for measuring objective ocular refraction and at least one geometrico-morphological parameter of an individual, said method comprising the following steps:
- activating at least one first target for stimulating the gaze (15) so as to stimulate the gaze of the individual in a first posture associated with a first proximity value (P1) and a first sighting axis,
- generating, by a lighting system, at least one lighting beam with the aid of a luminous source (8, 18, 28) directed toward the eyes of the individual in the first posture,
- obtaining, by an image capture system (6, 7), at least one first image acquisition of a part of the face surrounding the eyes of the individual, while masking the lighting system and the image capture system (6, 7) from the gaze with a splitting plate (13), or else by using a splitting plate (17) oriented in such a way that the image capture system (6, 7) is able to carry out an image acquisition of the wearer, while allowing the subject to observe the target (15) in transparency through the splitting plate,
- computing on the basis of said at least one first image acquisition on the one hand a first measurement of at least one geometrico-morphological parameter of the individual in the first posture and on the other hand a first measurement of objective ocular refraction of the individual in the first posture,
- activating at least one second target for stimulating the gaze (5) so as to stimulate the gaze of the individual in a second posture associated with a second proximity value (P2) and a second sighting axis,
- generating at least one lighting beam with the luminous source directed toward the eyes of the individual in the second posture,
- obtaining at least one second image acquisition of a part of the face surrounding the eyes of the individual with an optical return system (14, 16) disposed between on the one hand the second target for stimulating the gaze (5) and on the other hand the image capture system (6, 7) and the lighting system (8, 18, 28), the optical return system (14, 16) being adapted, in a determined orientation, to return the lighting beam toward the eyes of the individual looking at the second target (5) in a second posture,
- computing on the basis of said at least one second image acquisition on the one hand a second measurement of at least one geometrico-morphological parameter of the individual in the second posture and on the other hand a second measurement of objective ocular refraction of the individual in the second posture.

11. Measuring method according to Claim 10, in which said at least one geometrico-morphological parameter consists of at least one frame-fitting parameter for an individual wearing a spectacles frame (1), the frame-fitting parameter being selected from among the interpupillary distances, the lens-eye distance, the position of the center of rotation of the eye, the height of the eyes in relation to the lower edge of the frame.

12. Measuring method according to one of Claims 10 to 11, in which the first measurement of at least one geometrico-morphological parameter and the first measurement of objective ocular refraction of the individual in the first posture are sequential or simultaneous measurements.

13. Measuring method according to one of Claims 10 to 12, comprising an additional step of controlling the first posture and/or the second posture in such a way that the first sighting axis associated with the first posture and/or respectively the second sighting axis associated with the second posture, is included in a cone centered on the optical axis of the image capture system, said cone having a vertex angle of less than or equal to ten degrees.

14. Measuring method according to one of Claims 10 to 13, furthermore comprising a step of measuring an angle of lowering of the gaze of the individual.

15. Measuring method according to one of Claims 10 to 14, furthermore comprising a step of measuring at least one physiological parameter of vision of the individual wearing a spectacles frame (1) in the first and/or second posture, the physiological vision parameter being representative of strabismus, keratoconus or of cataracts or of behavioral parameters of the individual.

16. Measuring method according to one of Claims 10 to 14, in which the individual wearing a spectacles frame (1) equipped with lenses, the method comprises an additional step of correcting the ocular refraction measurement on the basis of at least one of the following values: the power correction value of the lenses or the value of the transmission coefficient of the lenses.
